Europäisches Patentamt

European Patent Office

Office européen des brevets

(11) Publication number: **0 451 550 A2**

# EUROPEAN PATENT APPLICATION

(21) Application number: **91104197.8**

(22) Date of filing: **19.03.91**

(51) Int. Cl.⁵: **C07K 7/06**, C07K 7/08, C07K 7/10, C07K 15/00, A61K 39/00, C12N 15/37, C12Q 1/70, G01N 33/569

(30) Priority: **20.03.90 EP 90105222**

(43) Date of publication of application:
**16.10.91 Bulletin 91/42**

(84) Designated Contracting States:
**AT BE CH DE DK ES FR GB GR IT LI LU NL SE**

(71) Applicant: **BEHRINGWERKE Aktiengesellschaft**
**Postfach 1140**
**W-3550 Marburg 1(DE)**

(72) Inventor: **Müller, Martin, Dr.**
**Husarenstrasse 14**
**W-6900 Heidelberg(DE)**
Inventor: **Gissmann, Lutz, Prof. Dr.**
**Pirolweg 1**
**W-6908 Wiesloch(DE)**

(74) Representative: **Klein, Otto, Dr. et al**
**Hoechst AG Zentrale Patentabteilung**
**Postfach 80 03 20**
**W-6230 Frankfurt am Main 80(DE)**

(54) Seroreactive epitopes of human Papillomavirus (HPV) 16 proteins.

(57) The present invention concerns seroreactive epitopes of the human Papillomavirus (HPV) 16 proteins E4, E6, E7 and L1 as well as peptides containing such epitopes and the use of these peptides for the production of a vaccine and a diagnostic kit.

This invention relates to seroreactive regions on the human Papillomavirus (HPV) 16 proteins E4, E6, E7 and L1.

This invention furthermore relates to vaccine containing peptides with sequences embracing those of such seropositive regions as well as diagnostic kits containing peptides with sequences embracing those of such seropositive regions.

The HPV16 is a type of the human Papillomavirus which has been first described in Proc. Natl. Acad. Sci., USA 80, 3813-3815 (1983).

The DNA-sequence and the genome organization of HPV 16 have been published in Virology 145, 181-185 (1985).

HPV16 is closely related not only to benign lesions of the anogenital tract but also to malignant cancer of the uterine cervix, penis and vulva. In addition HPV16 can also be found in genital scraps obtained from clinically asymptomatic individuals. Little is known about the immune response to infections by HPV16 and Papillomaviruses in general. In first experiments human sera obtained from STD patients, from patients suffering from cervical cancer as well as from healthy individuals were tested for the presence of antibodies directed against viral proteins. These proteins were expressed as fusions with different prokaryotic peptides linked to their N-terminus and used as antigens in Western-Blot experiments. This test is relatively tedious thus preventing a quantitative analysis of large serum collections. Moreover, due to the relatedness of the different Papillomavirus types, crossreactivity of antibodies cannot be excluded.

The object of the present invention is the identification of viral structures of HPV16 which may be used as tools in the prophylaxis, diagnosis and therapy of HPV16 dependent human diseases.

The knowledge of such domains is the prerequisite for the establishment of a peptide ELISA to be used for large scale screening of human sera.

The present invention therefore concerns:
- Seroreactive epitopes of the HPV16 protein E4 characterized by one of the following aminoacid sequences
  I. IPKPSPWAPKK
  II. KPSPWAPKKHRRLS;
- seroreactive epitopes of the HPV16 protein E6 characterized by one of the following amino acid sequences
  I. LSRHFMHQKRTAMFQDPQERPRKLPQ
  II. AMFQDPQERPRKLPQLCTELQTTIHDIILEC;
- seroreactive epitopes of the HPV16 protein E7 at the genomic region E7-221 characterized by one of the following amino acid sequences
  I. PTLHEYMLDLQPETTDLYCYEQ
  II. HEYMLDLQPET
  III. TLHEYMLDLQPETTD
  IV. EYMLDLQPETTDLY;
- seroreactive epitopes of the HPV16 protein E7 at the genomic region E7-107 characterized by one of the following amino acid sequences
  I. DEIDGPAGQAEPDRAHY
  II. GPAGQAEPDRAHYNI;
- seroreactive epitopes of the HPV16 protein L1 at the genomic region L1-809 characterized by the amino acid sequence
  PLLNKLDDTENASAYAANAGVDN;
- seroreactive epitopes of the HPV16 protein L1 at the genomic region L1-830 characterized by one of the following amino acid sequences
  I. ICTSICKYPDYIKMVSEPYGDSLFFYLRREQMFVRHLFNRAGTVGENVP DDLYIKGSGSTANLASSNYFPTPSGSMVTSDAQIFNKPY
- seroreactive epitopes of the HPV16 protein L1 at the genomic region L1-842 characterized by one of the following amino acid sequences
  I. KHTPPAPKEDDPLKK
  II. AIACQKHTPPAPKEDDPLKKYTFWEVNLKEKFSADLD
  III. LKKYTFWEVNLKEKFSADLDQF.

The present invention furthermore concerns:
- Peptides characterized in that they contain one or several of the above seroreactive epitopes;
- a vaccine characterized in that it contains one or several of the above peptides;
- a diagnostic kit for the identification of specific antibodies against HPV16 E4, E6, E7 or L1 proteins characterized in that it contains the above peptides;

- monoclonal antibodies characterized in that they have an affinity to the seroreactive epitopes of the human HPV16 proteins;
- a diagnostic kit which contains such monoclonal antibodies;
- a diagnostic agent containing such monoclonal antibodies for the identification of HPV16 specific proteins;
- the use of the above peptides for the production of the vaccine or the diagnostic kit.

In order to identify seroreactive epitopes within the proteins E4, E6, E7 and L1 of human Papillomavirus (HPV) type 16 the experimental approach described in Science 228, 1315-1317 (1985) was chosen. Short fragments of the viral genome were randomly cloned into the gene III protein of the single stranded DNA bacteriophage fd. Positive recombinants were identified by immunostaining with the appropriate antisera prepared against bacterial fusion proteins (Virology, 145, 181-185 (1985)) and the epitopes were determined by sequencing of the inserted DNA. Due to some background reactions probably depending upon the quality of the antiserum used only a proportion of the initially identified recombinants could be verified in subsequent tests after isolation and replating.

In the cases of repeatedly positive recombinants, however, this method proved to be highly specific as only sequences from such open reading frames (ORF) were found against which the antiserum was originally prepared and in most instances the respective sequences were identified repeatedly (see Table 3). Except for the E4 and L1 ORFs independent overlapping clones were found further underlining the reliability of the expression "library method". Additional support was provided by the fact that identical results were obtained with different antisera prepared in individual rabbits or even with sera derived from different species. The epitope 221 of the E7 protein was also identified by monoclonal antibodies. Moreover, synthetic peptides derived from the E7 epitope 107 and from the E4 epitope were found to react with a number of human sera.

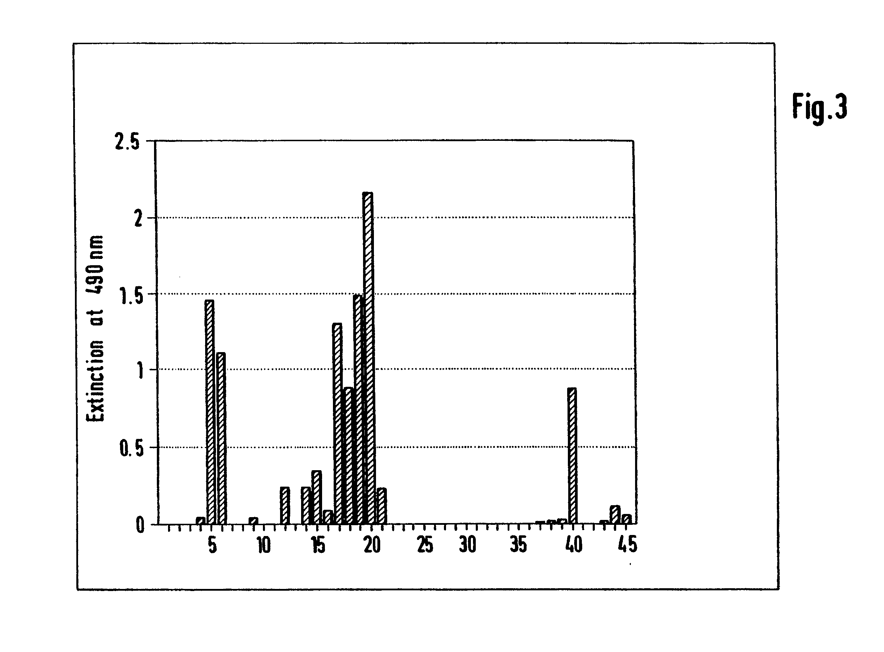

In case of the E4 ORF only one type of positive recombinants could be found. The epitope was confirmed, however, by an independent approach. Overlapping octapeptides spanning the entire ORF were synthesized and used to test an anti-E4 antiserum by ELISA. Four subsequent peptides covering a total of 14 aminoacids (Position 17-20) were shown to bind the immunoglobulins of the rabbit sera (Fig. 3). Peptide 17 is contained completely within the fd-derived epitope (Table 2) but is overlapping with peptide 20 by only one aminoacid. Therefore, it is not clear whether there are actually two adjacent epitopes within the E4 protein or whether peptide 20 is binding immunoglobulins in a sequence independent manner. In fact, such an unspecific binding is likely to be the case for the peptides 5/6 and 40 (Fig. 3) since the adjacent octapeptides overlapping by six amino acids are completely negative. In addition, only the synthetic peptide derived from the reactive region around peptide 20 but not from the other regions (Fig. 3) was found to react with the rabbit anti-serum by ELISA. Therefore unspecific binding due to some aberrant products synthesized onto the plastic pins could be the reason for the positive reaction at positions 5/6 and 40.

The methods used in the present invention solely permit the identification of linear but not of conformational epitopes. Therefore, it is an open question whether within the native proteins these domains are actually exposed to the immune system. In case of the E7 epitopes this appears to be rather likely since they are completely overlapping with or immediately adjacent to the putative E7 binding sites to the retinoblastoma protein and since the respective oligopeptide was shown to react with human sera. A similar observation was made for the peptide of the L1 ORF completely contained within the region L1-830 (Table 2).

The identified epitopes can be used as antigens for the screening of human sera for the presence of anti-HPV16 antibodies. It is an important question in this context whether within a given protein all epitopes have been identified. In the animal systems preincubation of the sera with synthetic peptides of the E4 and E7 epitopes prior to the reaction with the respective fusion proteins in Western-Blot experiments resulted in an almost complete loss of the signal (see also Fig. 3) indicating that actually no major epitope has been missed.

The identified epitopes of the different proteins of HPV16 should be useful as reagents in order to define the pattern of antibodies present in patients with HPV associated diseases as well as in healthy controls. Bacterial strains and phages: The filamentous phage derivative fuse 1 (fd-tet-J6); Science 228, 1315-1317 (1985), Gene 73, 305-318 (1988)) was used as expression system for HPV16 genomic DNA fragments in the unique PVUII site of fuse 1. For transformation with the fuse 1 vector the E.coli strain k802 (F galK2 galT22 metB1 supE44 hsdr2) (J. of Molec. Biol. 16, 118-133 (1966)) was used. The tetracyclin resistant colonies are producing bacteriophages which are not infectious for this strain because of its F-phenotype. For plating of the recombinant phages E. coli strain K91 (F+, derivative of K38; Virology 49, 45-60 (1972)) was used. Bacterial transformation was done according to J. of Molec. Biol. 166, 557-580 (1983). Insertion of a DNA fragment of the size 3n + 2 nucleotides without internal stop codons for translation is restoring a frameshift mutation within the gene III of fd which prevents production of infectious progeny.

Antisera: Polyclonal rabbit antisera prepared against an MS2 polymerase (Virology 145, 181-185 (1985)) or CII-HPV 16 E4 fusion protein or raised against a MS2 polymerase HPV16 E7 fusion protein were used. The E7 fusion part includes the amino acids from nt position 585 to 855, the E4 fusion parts the amino acids from nt position 3399-3617 on the HPV16 genome. Two different anti E7 monoclonal antibodies raised against the same fusion protein were used (E7 II and E7 IV, J. Gen. Virol. 68, 2933-2938 (1987)).

The anti-HPV16 E6 antiserum was prepared against an MS2 polymerase fusion containing the main part of the E6 ORF (nt position 110 to 556, Virology 145, 181-185 (1985)).

Two different polyclonal rabbit antisera prepared against the N-terminal (L1/1:nt position 5692 to 6819) and the C-terminal part (nt position 6819 to 7152) of HPV16 L1 fused to the N terminus of the M52 polymerase were used (Virology 145, 181-185 (1985)).

Preparing the HPV16 fuse 1 expression library: 5 g HPV16 plasmid DNA were sonicated for 120 sec to a fragment size of approximately 1 kb and further digested to about 300 bp with 0.02-2 units of DNase I for 10 min at 15 °C in the presence of 10 mM Mn++. The DNase I had been diluted and preincubated for 1 h in a buffer containing 50 mM Tris pH 8,0, 10 mM MnC12 and 0.1 mg/ml BSA.

To obtain blunt ends the DNA fragments were treated for 60 min at 15°C with 15 units of T4 polymerase and 10 units of E. coli DNA ligase and 100 M of each of the four deoxyribonucleotides. The DNA was then ligated into the unique PVUII site of fuse 1. Competent E. coli K802 (F-) were transformed with the ligated DNA and plated on LM tetplates. Approximately a total of 3x104 tetracyclin resistant phage producing colonies were obtained from eight different experiments. To obtain the recombinant phages the colonies were rinsed with LM. This amplification yielded in eight different libraries with a toal number of approximately 5 x 1012 infectious particles but resulted also in an underrepresentation of certain recombinants.

The phage suspensions were centrifuged and heated for 10 min at 65°C to remove the remaining bacteria. Unless indicated otherwise these amplified libraries were kept separately and used for further experiments.

Immunscreening: Between 2000 and 6000 phages were plated with 0.2 ml exponentially growing K91 cells in 3.5 ml 0.5% agarose containing 10 mM MgSO4 on minimal agar plates. Nitrocellulose replica were taken and incubated further on fresh minimal plates for 6 h at 37°C to enhance the signals. Afterwards the filters were blocked for 60 min in 10% nonfatty milk-PBS and then incubated overnight in 5% milk-PBS containing an 1 : 100 - 1 : 1000 dilution of HPV specific antisera (preadsorbed with sonicated K91 cells) or monoclonal antibodies. The filters were then washed 5 times for 5 min in PBS/0.1% Tween 20 and incubated for 3 h at room temperature with goat-anti-rabbit (or anti-mouse)-peroxidase antibodies (1 : 1000) in 5% nonfatty milk. After washing the filters were stained in 50 ml PBS containing 30 mg diaminobenzidine, 30 l H2O2 and 1.5 ml NiSO4. Finally the filters were washed in H2O for 30 min and dried on paper.

Preparation of single stranded DNA of fuse 1 recombinants: A protocol similar to previously described procedures was used (Proc. Nat. Acad. Sci. USA 74, 5463-5467 (1977)). 50 ml LM were inoculated with tet resistant E. coli K91 haboring the fuse 1 plasmid and incubated for 16 h at 37°C. The bacteria were then pelleted at 6000 rpm for 30 min. After adding 2 ml 40% PEG 6000 and 2 ml of 5 M sodiumacetat, pH 6.5 to the supernatant the phages were precipitated for 60 min at 0°C and then precipitated at 6000 rpm for 60 min. The pellet was resuspended in 0.3 ml TE. After two extractions with phenol the DNA was precipitated. Approximately 25% of such preparations were used for one sequencing reaction.

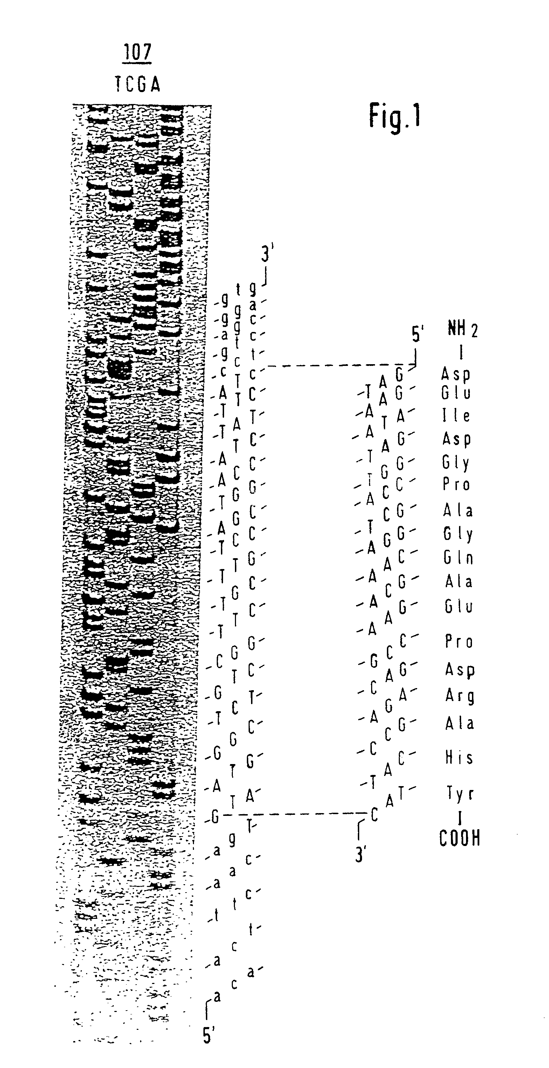

Sequencing: For DNA sequencing the standard USB ((United States Biochemicals) protocol (USB, 1987) was used and the universal primer was replaced by a 20mer oligonucleotide (5'-TCCAGACGTTAGTAAATGAA-3'). An example of a sequencing reaction is given in Fig. 1.

Peptide-synthesis: A set of overlapping peptides corresponding to the HPV16 E4 ORF was synthesized on the tips of polyethylene rods essentially following the strategy described in Proc. Nat. Acad. Sci. 82, 178 (1985); Proc. Natl. Acad. Sci. 81, 3998 (1985).

The polyethylene rods derivatized with beta-alanine were obtained from CRB, England. Deviating from the protocol recommended by Geysen the protein sequence was divided into octamer peptides overlapping by six residues and the synthesis was carried out using Fmoc-chemistry and in situ activation by BOP (Castro's reagent) (Tetrahedron Lett. 14, 1219 (1975)). Fmoc amino acid derivatives (6 micromoles) BOP and N-methyl morpholine solutions were distributed into polyethylene reaction trays (CRB) according to the respective peptide sequences being synthesized. All other reactions were carried out according to the CRB protocol. As positive control the peptide RPDYLDFA was synthesized together with the HPV16 specific peptides and tested with an appropriate antiserum by ELISA.

ELISA with octapeptides: Antibody detection on rod coupled octapeptides:

All tests were made on peptides covalently bound to the polyethylene pins on which they have originally been synthesized. Racks with 86 pins, fixed in a configuration that allows insertion into the wells of a microtitration tray were used. Incubations for ELISA were made while sticking the pins into the wells. The rods were washed with metha-nol an PBS and then blocked with O.25% gelantine, 0.1% Tween 20 in PBS for 2 h at 37°C followed by incubation with sera diluted 1 : 200 - 1 : 4000 in 0.125% gelantine, 0.05% Tween 20 for 1 h at 37°C. After washing with PBS/ 0.1% Tween 20 the pins were incubated for 1 h at 37°C with Protein A-peroxidase 1 : 4000 followed by a further washing and staining with tetramethylbenzidine (TMB; V.R. Holland et al. (1974), Tetrahedron 30, 3299-3302) for 15 min. Staining was terminated while lifting the rods out of the dye and adding of 100 l of 0.5 M H2SO4. The absorbtion was measured in an automatic ELISA reader. To remove the antibody-enzyme-complex after ELISA testing the pins were sonicated 1 h (waterbath, 30 W, 48 kHz) at 60°C in PBS/1% SDS/0.1% $\beta$-mercaptoethanol and finally washed with methanol. The efficiency of the disruption procedure was tested by ELISA using Protein A-peroxidase without any primary serum. The same peptides were used more than 40 times in subsequent ELISAs.

Short description of the Tables and Figures:

**Table 1:**

HPV16 DNA inserts representing the immunogenic region 221 on HPV16 E7. The E7-1 to E7-4 clones were identified by an anti E7 polyclonal rabbit antiserum. Group E7-4 clones are identical to those identified with the mouse monoclonal antibody (type II) described in J. Gen. Virol. 68, 2933-2938 (1987).

**Table 2:**

Immunogenic regions on the HPV16 proteins E4, E6, E7 and L1. The regions of E6 and E7 and L1-842 are represented in different fd clones. For the E4 protein the epitope identified by the overlapping octapeptides is also shown. The corresponding nucleotid positions of the first aminoacids and the last nucleotides of the last aminoacids are indicated. Clone 830, covering the immunogenic region L1-830 codes for 88 aminoacids. Its sequence is only presented in part. The number of individual clones representing the immunogenic regions are given in the last column.

**Fig. 1:**

Autoradiogram of a sequence reaction of fd clone 107 (see Table 2). The DNA sequence of the clone insert obtained with the sequencing primer, the corresponding coding sequences as well as the translated aminoacids are shown. Capital letters are indicating the HPV16 part of the DNA sequence.

**Fig. 2:**

Inhibition of immunostaining of MS2 polymerase - HPV16 E7 fusion protein with monoclonal antibodies by bacteriophages carrying the immunogenic region E7-221.

Western-Blot stripes stained with the monoclonal antibody type IV against HPV16 E7. The antibody was preadsorbed with preparations of different phage clones and incubated with the stripes containing a HPV16 E7-MS2 fusion protein. Only preparations of phage particles of clone 221 (lanes b and d; different concentrations of phage particles used for absorption; for clone numbers see Table 1) or clone 108 (lane e) can react with the monoclonal antibodies therefore preventing the reaction with the fusion protein on the stripes. Clones 209 (lanes c and f, different concentrations of phage particles) or 212 (g) do not interfere with staining of the protein. In lane a the antibodies were preabsorbed without phage particles, in lane h they were incubated with particles containing an insert not related to E7 sequences.

**Fig. 3:**

ELISA of overlapping octapeptides representing the HPV16 E4 ORF. The 45 peptides were incubated first with a polyclonal rabbit antiserum against HPV16 E7 followed by incubation with Protein Aperoxidase complex. The peptides were stained as described above and the Extinction was measured. A very similar pattern was obtained in four different experiments using two individual antisera.

## Example 1

### Identification of epitopes on HPV16-E7 protein using the fd expression library:

Approximately 25000 recombinant bacteriophages obtained from separately constructed HPV16-fd expression libraries were plated onto E. coli cells as described above. A total of 230 recombinants could be identified by screening with a polyclonal rabbit antiserum raised against an MS2 polymerase-HPV16 E7 fusion protein. Fifty-four of these recombinants were plated individually and rescreened, 31 of which remained positive after two to three subsequent screening steps and were further analysed: Bacteriophage particles were produced and the single stranded DNA prepared and used for sequencing as described above. The results are summarized in Table 1. All 31 phage clones were shown to contain HPV16-E7 specific sequences representing two different genomic regions (E7-221 and E7-107) on the E7 ORF.

Region E7-221 is represented by 22 clones which fall into four groups of different sizes. Group E7-1 consists of only one isolate of 68 nucleotides (bp 576 to bp 643). Groups E7-2, 3 and 4 are represented by 3, 9 and 9 clones, respectively, all of which are shorter than the E7-1 clone and overlap between the nucleotide position 589 to 618. Therefore, the smallest identi-fied epitope is represented by the amino acid sequence EYMLDLQPET.

Region E7-107 is represented by 9 clones of two different classes overlapping by 41 nucleotides and the resulting epitope has the sequence GPAGQAEPNRAHY (bp 679 to bp 717, Table 2).

## Example 2

### Identification of epitopes HPV16-E6 protein

Unlike in case of E7 it was not possible to identify E6 positive bacteriophages by screening an equivalent number of recombinants as before with a polyclonal rabbit anti-serum prepared against the MS2 polymerase-HPV16 E6 fusion protein. This may be explained by an underrepresentation of such clones in the phage library obtained from the original colonies.

To identify E6 reacting phages the immunoglobulin fraction of the anti-E6 rabbit serum was bound to Protein A-Sepharose. Approximately 1010 bacteriophages obtained as a pool of seven different HPV16 libraries constructed by individual ligation of sheared and DNase I digested HPV16 DNA of the fd plasmid were incubated with the Protein A-sepharose-immunoglobulin complexes. The specifically bound bacteriophages were eluted and plated on E. coli K91. Approximately 3000 plaques were obtained. For amplification the phages were suspended in medium and plated again.

In order to test the efficiency of the enrichment step identical numbers of recombinants eluted from the Protein A column were plated and hybridized with HPV16 DNA probes specific for the E6 (nt 24-654) or the E4 (nt 2714-3693) ORF. By comparison with the hybridisation of the original recombinants an approximately 20-fold enrichment of E6 positive phages were calculated. As the immunoreactive recombinants express only a part of the E6 protein (see below) it was assumed that such recombinants are even less prevalent in the original library and thus the factor of enrichment is higher. In fact approximately 300 positive signals were obtained when phages were screened with the same E6 antiserum used for the Protein A column. Fourteen of the positive recombinants were rescreened, 9 of which were finally analysed by DNA sequencing and shown to fall into two different classes representing one region of the HPV16 E6 ORF. The overlapping sequence (bp 101 to 145) is coding for the epitope AMFQDPQERPRKLPQ.

## Example 3

### Identification of epitopes on HPV16-L1 protein

Within the HPV16 L1 protein three different immunogenic regions were identified. 20000 recombinant bacteriophages of one HPV16 library were screened with a rabbit antiserum raised against the N-terminal part of the HPV16 L1 ORF (bp 5695 to 6818). Twenty-five out of 80 positive signals were rescreened and eleven of them were sequenced. Three clones were shown to contain an identical fragment of the HPV16 L1 ORF, i.e. nt position 5998-6066 coding for the peptide PLLNKLDDTENSASASAYAANAGVDN (L1-809; Table 2). All the other eight clones contained an insert (nt position 6307-6570) coding for an 88 aminoacids long peptide I(CTSICKYPD----SDAQIFNKPY; L1-830).

Screening of 8000 recombinants of the same HPV16 library with a polyclonal rabbit antiserum raised against the C-terminal part of HPV16 L1 (bp 6818 to 7152) resulted in 52 positive recombinants. Six of them were confirmed by repeated rescreening and their inserts sequenced. All clones were shown to belong to the same immunogenic region (L1-842, Table 2). The insert of two clones (842, nt 6922-6966) codes for the peptide KHTPPAPKEDDPLKK which is overlapping by three aminoacids with the insert of clone 877 (nt 6958-7023) coding for the peptide LKKYTFWEVNLKEKFSADLDQF. A third group of recombinants (905; nt 6907-7017) represented by four clones is overlapping with the clones 842 and 877 and is coding for the 37mer peptide (AIACQKHTPPAPKEDDPLKKYTFWEVNLKEKFSADLD). Since the common sequence of all three clones consists of only three aminoacids (LKK) there are probably two independent binding sites within the region L1-842 (nt 6907-7023) although the existence of such a short epitope cannot be excluded.

## Example 4

### Identification of epitopes on HPV16-E4 protein

To identify E4 epitopes the same library as for L1 was used. By screening with an anti-E4 serum prepared against an MS2-fusion-protein 28 recombinants were identified. Three of them were analysed by sequencing and were shown to contain the HPV16 E4-ORF specific insert (nt position 3422 to 3456) corresponding to the peptide IPKPSPWAPKK.

### Example 5

### Identification of the binding site of an anti E7 monoclonal mouse antibody:

As described for the identification of E6 specific epitopes 1010 phages obtained from seven different HPV16 fd-libraries were bound to Protein A-Sepharose-IgG (mouse monoclonal) complexes. After elution approximately 300 recombinants were obtained, plated and suspended in LM. 500 recombinants of this sublibrary were screened with the anti HPV16 E7 monoclonal antibody de-signated II. Two of the clones were sequenced after three steps of rescreening. Both clones were shown to contain an insert identical with the insert of clones of group 4 of the immunogenic region 221 of the HPV16 E7 ORF (Table 1).

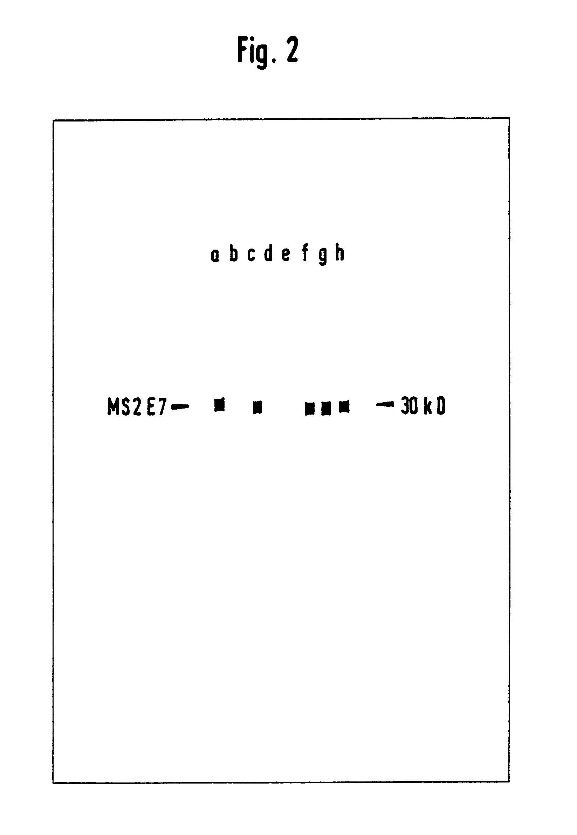

In order to determine whether all different clones of the immunogenic region 221 bind HPV16 anti-E7 monoclonal antibodies competition assays were performed. MS2 polymerase-E7 fusion protein on Western-Blot strips was stained with two different monoclonal antibodies (E7 II and E7 IV, J. Chem. Virol. 68, 2933-2938 (1987)). Purified recombinant bacteriophage particles carrying the group 1 to 4 epitopes (Table 1) were used for preadsorption of the monoclonal antibodies before immunostaining of the Western-Blot. No difference was observed when the monoclonal antibody E7 IV was used for staining. As shown in Fig. 2 only bacteriophage particles of group 1 and 4 prevent the binding of the monoclonal antibody E7 II to the E7 protein. Therefore, it was concluded that there is at least one additional binding site for antibodies adjacent to the epitope EYMLDLQPET of the immunogenic region E7-22I described above.

### Example 6

### Identification of immunogenic regions on the HPV16 E4 protein by testing overlapping peptides:

Fourty-five octapeptides representing the HPV16 E4 ORF were synthesized on polyethylene pins as described above.

The peptides were overlapping by six aminoacids thus peptide number one represents aminoacids 1 to 8 of the HPV16 E4 ORF, peptide number two encompasses aminoacids 3 to 10 and so on. The peptides were incubated with the polyclonal rabbit antiserum raised against an MS2-HPV16 E4 fusion protein mentioned above. Rabbit immunoglobulins were detected by incubation with Protein Aperoxidase complexes followed by staining with TMB as described in Materials and Methods. As shown in Fig. 4 a major cluster of antibody binding peptides was obtained with the rabbit antiserum. It includes the peptide KPSPWAPKKHRRLS of the HPV16 E4 ORF. This sequence is partially included in the reactive region identified by the immunoscreening (Table 3). Testing of the peptides with an independently produced rabbit antiserum prepared against an CII-HPV16 E4 fusion protein resulted in a very similar pattern compared to that obtained with the anti-MS2 E4 antiserum (data not shown). Incubation with rabbit control sera or with Protein Aperoxidase alone gave no significant signals. Additional signals obtained with single peptides (e.g. peptide no. 40; see Fig. 3) are very likely not indicating a specific immunoglobulin binding epitope since in contrast to the neptide kPSPWAPKKHRLS synthetic oligopeptides derived from these regions fail to react with the antisera in ELISA (data not shown).

Table 1

Immunogenic Region 221 on the HPV 16 E7 Protein

---

E7-1 (1 clone):

5'-A CCT ACA TTG CAT GAA TAT ATG TTA GAT TTG CAA CCA GAG ACA ACT GAT CTC TAG TGT TAT GAG CAA T-3'

---

E7-2 (3 clones):

5'-G CAT GAA TAT ATG TTA GAT TTG CAA CCA GAG ACA A-3'

---

E7-3 (9 clones):

5'-T ACA TTG CAT GAA TAT ATG TTA GAT TTG CAA CCA GAG ACA ACT GAT C-3'

---

E7-4 (9 clones):

5'-T GAA TAT ATG TTA GAT TTG CAA CCA GAG ACA ACT GAT CTC TAC T-3'

---

EP 0 451 550 A2

Table 2

<hr>

Immunogenic region E4-815 (HPV 16 E4):

| 815 | bp3423-I P K P S P W A P K K-bp3455 | 3 |
| overlapping peptides: | K P S P W A P K K H R R L S | |

<hr>

Immunogenic region E6-670 (HPV 16 E6):

| 670 | bp68-L S R H F M H Q K R T A M F Q D P Q E R P R K L P Q-bp145 | 4 |
| 676 | bp101-A M F Q D P Q E R P R K L P Q L C T E L Q T T I H D I I L E C-bp193 | 5 |

<hr>

Immunogenic region E7-221 (HPV 16 E7):

| 221 | bp577 -P T L H E Y M L D L Q P E T T D L Y C Y E Q-bp642 | 1 |
| 209 | bp586 -H E Y M L D L Q P E T-bp618 | 3 |
| 212 | bp580 -T L H E Y M L D L Q P E T T D-bp624 | 9 |
| 108 | bp589 -E Y M L D L Q P E T T D L Y-bp630 | 9 |

<hr>

Immunogenic region E7-107 (HPV 16 E7):

| 107 | bp667-D E I D G P A G Q A E P D R A H Y-bp717 | 6 |
| 710 | bp679-G P A G Q A E P D R A H Y N I-bp723 | 3 |

<hr>

TO BE CONTINUED

Table 2, continued:

Immunogenic region L1-809 (HPV 16 L1):

809  bp5998-P L L N K L D D T E N A S A Y A A N A G V D N-bp6066    3

Immunogenic region L1-830 (HPV 16 L1):

830  bp6307-I C T S I C K Y P N----------S N A Q I F N K P Y-bp6570    8

Immunogenic region L1-842 (HPV 16 L1):

842      bp6922-K I I T P P A P K E D D P L K K-bp6966    2

905  bp6907-A I A C Q K H T P P A P K E D D P L K K Y T F W E V N L K E K F S A D L D-bp7017    4

877           bp6958-L K K Y T F W E V N L K E K F S A D L D Q F-bp7023    1

## Claims

1. Seroreactive epitopes of the HPV16 protein E4 characterized by one of the following aminoacid sequences

I. IPKPSPWAPKK

II. KPSPWAPKKHRRLS.

2. Sercreactive epitopes of the HPV16 protein E6 characterized by one of the following amino acid sequences

I. LSRHFMHQKRTAMFQDPQERPRKLPQ

II. AMFQDPQERPRKLPQLCTELQTTIHDIILEC.

3. Seroreactive epitopes of the HPV16 protein E7(-221) characterized by one of the following amino acid sequences

I. PTLHEYMLDLQPETTDLYCYEQ

II. HEYMLDLQPET

III. TLHEYMLDLQPETTD

IV. EYMLDLQPETTDLY.

4. Seroreactive epitopes of the HPV16 protein E7(-107) characterized by one of the following amino acid sequences

I. DEIDGPAGQAEPDRAHY

II. GPAGQAEPDRAHYNI.

5. Seroreactive epitopes of the HPV16 protein L1(-809) characterized by the amino acid sequence

PLLNKLDDTENASAYAANAGVDN.

6. Seroreactive epitopes of the HPV16 protein L1(-830) characterized by one of the following amino acid sequences

I. ICTSICKYPN--SNAQIFNKPY

II. ICTSICKYPDYIKMVSEPYGDSLFFYLRREQMFVRHLFNRAGTVGEN VPDDLYIKGSGSTANLASSNYFPTPSGSMVTSDAQIFNKPY

7. Seroreactive epitopes of the HPV16 protein L1(-842) characterized by one of the following amino acid sequences

I. KHTPPAPKEDDPLKK

II. AIACQKHTPPAPKEDDPLKKYTFWEVNLKEKFSADLD

III. LKKYTFWEVNLKEKFSADLDQF

8. Peptides characterized in that they contain one or several of the seroreactive epitopes according to claims 1 through 7.

9. Vaccine characterized in that it contains one or several of the peptides according to claim 8.

10. Diagnostic kit for the identification of specific antibodies against HPV16 E4, E6, E7 or L1 proteins characterized in that it contains peptides according to claim 8.

11. Monoclonal antibodies characterized in that they have an affinity to the seroreactive epitopes of the human HPV16 proteins according to claims 1 to 7.

12. Diagnostic kit which contains the monoclonal antibody according to claim 11.

13. Diagnostic agent according to claim 12 for the identification of HPV16 specific proteins.

14. Use of peptides according to claim 8 for the production of the vaccine of claim 9 or the diagnostic kit of claim 10.

**Claims for the following Contracting States: ES, GR**

1. Process to manufacture a diagnostic kit for the identification of specific antibodies against HPV16 E4, E6, E7 or LI proteins characterized in that peptides containing one or several of the epitopes

```
I.    IPKPSPWAPKK
                                              (E4)
II.   KPSPWAPKKHRRLS.


I.    LSRHFMHQKRTAMFQDPQERPRKLPQ
                                              (E6)
II.   AMFQDPQERPRKLPQLCTELQTTIHDIILEC.


I.    PTLHEYMLDLQPETTDLYCYEQ
II.   HEYMLDLQPET
III.  TLHEYMLDLQPETTD
                                              (E7)
IV.   EYMLDLQPETTDLY.                         -221


I.    DEIDGPAGQAEPDRAHY
                                              (E7)
II.   GPAGQAEPDRAHYNI.                        -107


      PLLNKLDDTENASAYAANAGVDN.                (L1)
                                              -809


I.    ICTSICKYPN--SNAQIFNKPY
II.   ICTSICKYPDYIKMVSEPYGDSLFFYLRREQMF
                                              (LI)
      VRHLFNRAGTVGENVPDDLYIKGSGSTANLASS       -830
      NYFPTPSGSMVTSDAQIFNKPY


I.    KHTPPAPKEDDPLKK
II.   AIACQKHTPPAPKEDDPLKKYTFWEVNLKEKFS
                                              (LI)
      ADLD                                    -842
III.  LKKYTFWEVNLKEKFSADLDQF
```

are coated to a solid phase such that antibodies directed to said epitopes are detectable by immunoassay techniques.

2. Process according to claim 1, characterized in that the diagnostic kit is an ELISA.

3. Process to manufacture a diagnostic kit to detect specific epitopes of HPV16 characterized in that one or more of the epitopes used in claim 1 are taken to produce monoclonal antibodies which are subsequently coated to a solid phase such that said specific epitopes are detectable.

4. Process to manufacture a vaccine against HPV16 characterized in that one or more of the peptides used in claim 1 are mixed with suitable adjuvants and carrier.

## 107

TCGA

# Fig.1

3'

5'    NH₂
      |
G     Asp
C
A     Glu
T     Ile
C     Asp
G     Gly
C     Pro
G     Ala
C     Gly
G     Gln
C     Ala
G     Glu

      Pro
C     Asp
A     Arg
G     Ala
C     His
T     Tyr
      |
      COOH

3'

5'

# Fig. 2

Fig.3